# EUROPÄISCHE PATENTSCHRIFT

(11) **EP 0 480 872 B1**
(45) Veröffentlichungstag und Bekanntmachung des Hinweises auf die Patenterteilung: **09.11.1994**
(21) Anmeldenummer: 91810719.4
(22) Anmeldetag: 06.09.1991
(51) Int. Cl.: A61F 2/36, A61F 2/30

(54) **Femurkopfprothese**
Femoral head prosthesis
Prothèse de tête fémorale

(30) Priorität: 10.10.1990 CH 3263/90
(43) Veröffentlichungstag der Anmeldung: 15.04.1992
(73) Patentinhaber: SULZER Medizinaltechnik AG, 8404 Winterthur (CH)
(72) Erfinder: Horber, Willi, CH-8005 Zürich (CH); Koch, Rudolf, CH-8267 Berlingen (CH)
(74) Vertreter: Hammer, Bruno, Dr.

(56) Entgegenhaltungen:
- EP-A- 0 145 938
- DE-A- 3 122 730
- DE-A- 3 904 528
- FR-A- 2 425 237
- US-A- 4 650 489

## Beschreibung

Die Erfindung betrifft eine Femurkopfprothese, die aus einem Schaft mit Hals, mit Schaftrand und mit einem aufgesetzten Kugelkopf besteht, wobei der Schaft distal in Richtung der Schaftachse in mehreren einseitig eingespannten Biegefedern endet, welche von einem dünnwandigen und deformierbaren Hüllkörper eingeschlossen sind, während der Hüllkörper mit dem Schaftrand verbunden ist.

Bei der zementfreien Verankerung von Femurkopfprothesen in der Markraumhöhle eines Femurknochens, die nach innen konisch zulaufend nachbearbeitet wurde, ist für die Primärverankerung eine sehr genaue Formgebung der Markraumhöhle notwendig. Ausserdem sollte die Prothese für die in der transversalen Ebene auftretenden Biegekräfte eine ähnliche Elastizität wie das stützende Knochengewebe aufweisen, um stossartige Belastungen ohne lokale Spannungsspitzen zum Knochengewebe abzubauen.

Die FR-B-2 591 885 zeigt einen, in der transversalen Ebene gespaltenen Schaft aus Memory-Metall, der bei Temperaturen unter der des menschlichen Körpers mindestens einen Schlitz aufweist, der sich nach dem Einbringen des Schaftes bei Körpertemperatur nach anterior und posterior aufzuweiten sucht und den Schaft gegen das Knochengewebe verspannen soll. Aehnlich zeigt die FR-B-2 549 717 einen Schaft mit mehreren Zungen, die zur besseren Verankerung des Schaftes beim Einbringen vorgespannt werden.

Bei beiden Ausführungen wächst Knochengewebe in die offenen Schlitze ein und verhindert eine Rückdeformation, um den Schaft im Fall einer Reoperation ziehen zu können. Im weiteren muss die Steifigkeit für die verspannten Schaftteile so hoch gewählt werden, dass unter einer stossartigen Gewichtslast und unter der konischen Zentrierwirkung der Markraumhöhle für das Knochengewebe tolierbare Verschiebungen der Schaftoberfläche in Richtung der Schaftachse stattfinden.

In der DE-A-31 22 730 sind Schäfte für Femurkopfprothesen gezeigt, die proximal einen starren Zylinder aufweisen, an dem ein Sack befestigt ist, welcher sich gegen distal erstreckt. Aus dem Zylinder ragen elastische Speichen über die ganze Schaftlänge in den Sack hinein, wobei die Zwischenräume zwischen den Speichen und auch zum Sack hin mit magnetisierbaren Pulver, Kugeln oder Brocken angefüllt sind, um durch Entmagnetisieren sowie anschliessendes Schütteln unter Schwerkraft, den Schaft zum Anliegen in der Knochenaushöhlung zu bringen und diese Anliegeposition durch Magnetisieren des Pulvers, der Kugeln oder der Brocken zu fixieren. Ein Nachteil dieser Methode ist, dass das Pulver, die Kugeln oder Brocken bei ungenügender Magnetkraft wie eine Flüssigkeit dem Druck der Speichen ausweichen können und somit die Winkellage der Schaftachse im Zylinderteil ungewollt verändern können.

In der FR-A-24 25 237 sind Speichen oder Blattfedern gezeigt, die sich über einen Grossteil der Schaftlänge erstrecken, wobei sie am einen Ende des Schaftes starr miteinander verbunden sind, während am anderen Ende des Schaftes ein Teil der Speichen oder Blattfedern mit einer Führungsbüchse starr verbunden ist, in welcher der andere Teil der Speichen oder Blattfedern in axialer Richtung verschiebbar gelagert ist. Diese Ausführung setzt die Biegesteifigkeit des Schaftes herab, ohne jedoch die Verankerung selbst zu verbessern. Im weiteren kann an den Führungsbüchsen Abrieb entstehen, der in das Knochengewebe gelangt.

Hier schafft die Erfindung Abhilfe. Sie hat die Aufgabe, eine dauerhafte Verbindung vom Prothesenschaft zum Knochengewebe zu schaffen. Diese Aufgabe wird mit den Kennzeichen des unabhängigen Anspruchs 1 gelöst. Die Erfindung hat den Vorteil, dass das Knochengewebe im nicht belasteten Zustand unter einer definierten Vorspannung in grossen Oberflächenbereichen vorgespannt ist, während bei grösseren stossartigen Lasten eine Reibungsdämpfung an den Berührungspunkten zwischen den Blattfedern einsetzt. Im unteren Schaftbereich verschieben sich die medialen Blattfedern tiefer längs der Schaftachse und kompensieren mit ihren angeschrägten Stirnflächen teilweise den Querschnittsverlust im Schaft, der sich durch eine weitere Verengung der Schlitze ergibt.

Weitere Vorteile der Erfindung sind darin zu sehen, dass der konische Schaftteil unter einer definierten mittleren Mindestvorspannung anliegt, solange keine Last auftritt, dass beim Auftreten einer Last durch die innere Deformation des Schaftes eine wesentlich grössere Drucksteifigkeit entsteht, und dass mit der inneren Deformation Reibungsarbeit geleistet wird, die Stossenergie abbaut. Die abhängigen Ansprüche 2 bis 5 beziehen sich auf vorteilhafte Weiterbildungen der Erfindung.

Im folgenden wird die Erfindung anhand eines Ausführungsbeispiels beschrieben. Es Zeigen:
- Fig. 1: Von posterior die Ansicht einer Femurkopfprothese, bei der der Hüllkörper weggeschnitten ist, im nicht eingebauten, unbelasteten Zustand;
- Fig. 2: die Seitenansicht einer Femurkopfprothese gemäss Fig. 1 in transversaler Richtung;
- Fig. 3: von posterior die Ansicht einer Femurkopfprothese, bei der der Hüllkörper weggeschnitten ist, im eingebauten und belasteten Zustand, und
- Fig. 4: das nicht lineare Kraft-Wegdiagramm der Oberfläche im unteren Schaftbereich für eine Druckkraft F₁ in transversaler Richtung gemäss Fig. 3.

In den Figuren ist eine Femurkopfprothese gezeigt, die aus einem Schaft mit Hals, mit einem Schaftrand und mit einem aufgesetzten Kugelkopf besteht. Der Schaft endet distal in Richtung der Schaftachse in mehreren einseitig eingespannten Blattfedern, welche von einem dünnwandigen und deformierbaren Hüllkörper eingeschlossen sind. Der Hüllkörper ist mit dem Schaftrand verbunden und schliesst das eingeschlossene Volumen gasdicht ab. Die Blattfedern sind durch Schlitze voneinander getrennt. Die Tiefe der Schlitze gegen proximal lässt einen schmalen ungeschlitzten Jochteil stehen. Im implantierten Zustand ohne Gewichtsbelastung sind die Blattfedern vom Einsetzen in die vorbearbeitete Markraumhöhle so nach innen deformiert, dass sie mit den Nachbarblattfedern gemeinsame Berührungspunkte aufweisen. Beim Auftreten einer stossartigen Gewichtskraft erfolgt eine leichte Biegung im Jochteil und innerhalb des Hüllkörpers eine leichte Verschiebung der Biegefedern zueinander, die eine Dämpfung und einen nicht linearen Anstieg der Druckkraft zur Folge hat.

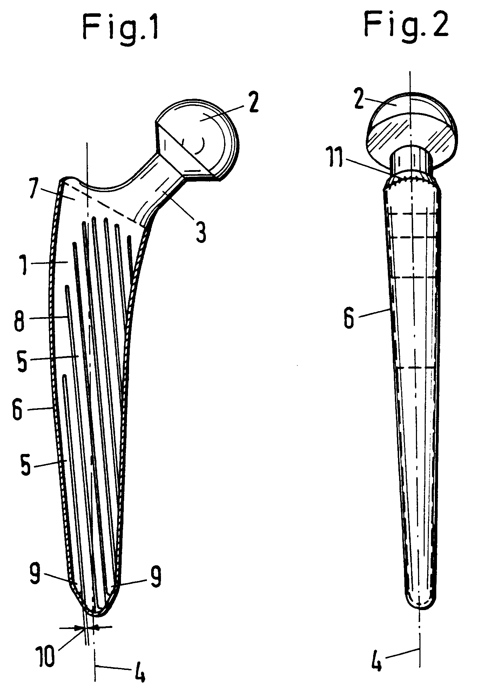

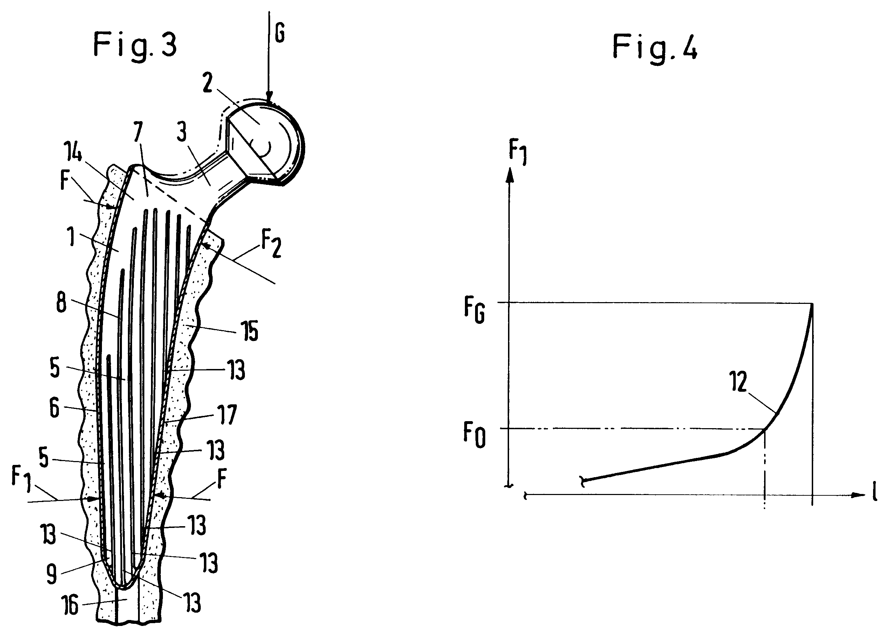

Fig. 1 zeigt einen Schaft mit Blattfedern 5, die durch Schlitze 8 getrennt sind, welche in zueinander und annähernd zur Schaftachse 4 parallelen und in mediolaterialer Richtung versetzten Ebenen liegen. Die freien Enden 9 weisen im Vergleich zu einem vollen Schaft eine geringe Biegesteifigkeit in medio-lateraler Richtung auf. Beim Einbringen in die vorbearbeitete Markraumhöhle 16 eines Femurknochens (Fig. 3) werden zunächst die äusseren Blattfedern um eine Schlitzbreite 10 deformiert, bis sie die innen anschliessenden Blattfedern 5 an einem Berührungspunkt 13 berühren, wobei letztere bei einer Fortsetzung der Deformation ebenfalls Widerstand leisten. Bis zur Aufhebung aller Schlitzbreiten 10 im unteren Schaft durchläuft eine Druckkraft F₁ - wie in Fig. 4 gezeigt - den ersten Teil einer nicht linearen Charakteristik 12 für eine überproportionale Zunahme der Druckkraft bis zu einem Betrag Fₒ. Der Hüllkörper 6, der vorzugsweise gekreppt ausgeführt ist, vermag der Deformation der Biegefedern zu folgen. Er ist mit dem Schaftrand 7 durch eine Schweissnaht 11 gasdicht verbunden, um Abriebpartikel vom Knochengewebe 15 fernzuhalten. Die Steifigkeit der Blattfedern und die Schlitzbreiten 10 sind so gewählt, dass mit dem sich gegenseitigen Abstützen der Blattfedern die ideale Vorspannkraft Fₒ für den nicht belasteten Zustand erreicht wird.

In Fig. 3 ist die unter einer Gewichtskraft G auftretende Deformation durch eine verschobene Lage am Kugelkopf 2 angedeutet. Bei einem starren Schaft würden ausgeprägte Reaktionen gegen die Angriffsrichtung von F₁ und F₂ auftreten, um ein durch G erzeugtes Biegemoment zu kompensieren. Gleichzeitig würde eine aus Normalkräften F und eine aus Reibungskräften an der Schaftoberfläche zusammengesetzte Reaktion entstehen, die in Betrag gleich und in Richtung parallel verschoben und entgegengesetzt zu G ausfällt. Für den geschlitzten Schaft reichen die Schlitze 8 soweit gegen proximal in den Schaftkörper hinein, dass als ungeschlitzter Bereich ein biegefähiges Joch 14 zum Hals 3 stehen bleibt. Dieses Joch 14 erfährt unter der Gewichtskraft G eine Auslenkung, die es auf die Biegefedern als Bewegung in Richtung der Schaftachse weitergibt, wobei die gegen medial angeordneten Biegefedern mit ihrer angeschrägten Stirnfläche 17 gegenüber den Nachbarbiegefedern tiefer abwärts gestossen werden und den durch Einfederung verursachten Verlust an Querschnitt teilweise durch die konische Vergrösserung mit den Stirnflächen 17 kompensieren. Die messbare Einfederung bei einer Kraft F fällt kleiner aus, d.h. das System wirkt steifer. Mit wachsender Deformation 1 steigt daher die Kraft F₁ überproportional von Fₒ auf F_{G} an. Wegen dieses nicht linearen Anstiegs der Federkraft sind die Bewegungen zwischen dem Hüllkörper 6 und dem eingewachsenen Knochengewebe gering. Wesentlich ist, dass die Stirnflächen 17 entlang dem Hüllkörper - wenn auch in kleinem Ausmass - gleiten können.

Die Blattfedern 5, die bei stossartigen Gewichtsbelastungen mit Ueberwindung der Haftreibung aneinander gleiten und reiben, absorbieren Stossenergie und stellen ein Dämpfungsglied zum Femurknochen dar.

## Patentansprüche

1. Femurkopfprothese bestehend aus einem Schaft (1) mit Hals (3), mit Schaftrand (7) und mit einem aufgesetzten Kugelkopf (2), wobei der Schaft (1) distal in Richtung der Schaftachse (4) in mehreren einseitig eingespannten Biegefedern (5) endet, welche vom einem dünnwandigen und deformierbaren Hüllkörper (6) eingeschlossen sind, während der Hüllkörper (6) mit dem Schaftrand (7) verbunden ist, dadurch gekennzeichnet, dass die Biegefedern (5) als Blattfedern ausgeführt sind, die durch Schlitze (8) voneinander getrennt sind, welche in zueinander parallelen und in medio-lateraler Richtung versetzten Ebenen liegen und dass der Hüllkörper (6) das eingeschlossene Volumen gasdicht abschliesst.

2. Femurkopfprothese nach Anspruch 1, dadurch gekennzeichnet, dass die Schlitze (8) beim Fortschreiten in einer ausgewählten Richtung in der transversalen Ebene eine geringere Schlitzbreite (10) als der jeweils da vor liegende Schlitz aufweisen.

3. Femurkopfprothese nach Anspruch 1 oder 2, dadurch gekennzeichnet, dass die Schlitze (8) so tief gegen proximal eingeschnitten sind, dass bei üblichen Gewichtskräften G in einem verbleibenden nicht geschlitzten Jochteil (14) zum Hals (3) eine elastische Biegedeformation auftritt, auf Grund derer eine Blattfeder (5) gegenüber einer lateral davorliegenden Blattfeder (5) eine Abwärtsverschiebung in Richtung der Schaftachse (4) erfährt.

4. Femurkopfprothese nach einem der Ansprüche 1 bis 3, dadurch gekennzeichnet, dass die Blattfedern (5), die gegen medial angeordnet sind, eine in der Kontur des Schaftes angeschrägte Stirnfläche (17) aufweisen, welche bei einer Verschiebung gegen distal zu einer Querschnittsvergrösserung am Schaft führt.

5. Femurkopfprothese nach einem der Ansprüche 1 bis 4, dadurch gekennzeichnet, dass der Hüllkörper (6) im Bereich der Blattfedern gekreppt ausgeführt ist, um den Bewegungen der Blattfedern (5) bei Belastung zu folgen.

## Claims

1. A femur head prosthesis consisting of a shaft (1) having a neck (3), a shaft edge (7) and a ball end (2) on top, wherein the shaft (1) ends distally in the direction of the shaft axis (4) in several bending springs (5) fixed at one end, which are enclosed by a thin-walled and deformable envelope member (6), while the envelope member (6) is connected to the shaft edge (7),
characterised in that the bending springs (5) are constructed as leaf springs, which are separated from one another by slits (8), which lie in planes which are parallel to one another and offset in the mediolateral direction;
and in that the envelope member (6) seals the enclosed volume in gas-tight manner.

2. A femur head prosthesis according to claim 1,
characterised in that the slits, when progressing in a selected direction in the transverse plane, have a smaller slit width (10) than the respectively preceding slit.

3. A femur head prosthesis according to claim 1 or 2,
characterised in that the slits (8) are cut with such a depth in the proximal direction that with normal weight loads G an elastic bending deformation occurs in a remaining unslit yoke part (14) to the neck (3), as a result of which a leaf spring (5) undergoes a downward displacement in the direction of the shaft axis (4) when compared with a leaf spring (5) laterally in front of said spring.

4. A femur head prosthesis according to one of the claims 1 to 3,
characterised in that the leaf springs (5), which are disposed medially, have an end surface (17) chamfered in the contour of the shaft, which, during a displacement in the distal direction, leads to an enlargement of the cross-section at the shaft.

5. A femur head prosthesis according to one of the claims 1 to 4,
characterised in that the envelope member (6) is crinkled in the region of the leaf springs so that it can follow the movements of the leaf springs (5) when loaded.

## Revendications

1. Prothèse de tête de fémur, se composant d'une tige (1) comportant un col (3), un bord (7) de tige et une tête sphérique rapportée (2), la tige (1) s'achevant du côté distal, dans la direction de son axe (4), en plusieurs ressorts de flexion (5) fixés d'un côté et enfermés dans un corps d'enveloppement (6) à cloison mince qui est déformable, tandis que le corps d'enveloppement (6) est relié au bord (7) de tige, caractérisée en ce que les ressorts de flexion (5) sont conformés en ressorts à lames de ressort qui sont séparés par des fentes (8) qui sont situées dans des plans parallèles et décalés les uns par rapport aux autres dans la direction médio-latérale et en ce que le corps d'enveloppement (6) ferme de façon étanche aux gaz le volume occlus.

2. Prothèse de tête de fémur selon la revendication 1, caractérisée en ce que les fentes (8) observées en progressant dans un sens sélectionné dans le plan transversal ont une largeur (10) qui est inférieure à celle de la fente qui la précède.

3. Prothèse de tête de fémur selon la revendication 1 ou 2, caractérisée en ce que les fentes (8) sont taillées vers le côté proximal à une profondeur telle que, sous des forces pondérales usuelles G, il se produit dans une partie de portée subsistante (14) non fendue et aboutissant au col (3) une déformation élastique de flexion en raison de laquelle une lame de ressort (5) subit par rapport à une lame de ressort latérale (5) qui la précède un déplacement vers le bas et vers l'axe (4) de la tige.

4. Prothèse de tête de fémur selon l'une des revendications 1 à 3, caractérisée en ce que les ressorts à lames (5) qui sont disposés vers le côté médial, ont une surface extrême oblique (17) qui épouse le contour de la tige et qui, lorsqu'elles sont déplacées vers le côté distal, provoque un accroissement de section au niveau de la tige.

5. Prothèse de tête de fémur selon l'une des revendications 1 à 4, caractérisée en ce que le corps d'enveloppement (6) est crêpé dans la région des ressorts à lames afin de suivre sous charge les mouvements des ressorts à lames (5).
